(19) Europäisches Patentamt
European Patent Office
Office européen des brevets

(11) Numéro de publication : **0 452 170 A1**

(12) **DEMANDE DE BREVET EUROPEEN**

(21) Numéro de dépôt : **91400688.7**

(51) Int. Cl.[5] : **C07D 211/70, A61K 31/445**

(22) Date de dépôt : **13.03.91**

(30) Priorité : **14.03.90 IT 1967590**

(43) Date de publication de la demande :
**16.10.91 Bulletin 91/42**

(84) Etats contractants désignés :
**AT BE CH DE DK ES FR GB GR IT LI LU NL SE**

(71) Demandeur : **ROUSSEL-UCLAF**
**35, Boulevard des Invalides**
**F-75007 Paris (FR)**

(72) Inventeur : **Toja, Emilio**
**Via Plezzo 80**
**Milano (IT)**
Inventeur : **Bonetti, Carla**
**Via Beccalino**
**Fontanella (Bergamo) (IT)**
Inventeur : **Barzaghi, Fernando**
**Via Monteverdi 21**
**I-20052 Monza - Milan (IT)**
Inventeur : **Galliani, Giulio**
**13, Via Silva**
**1 Monza (IT)**

(74) Mandataire : **Tonnellier, Marie-José et al**
**111, route de Noisy B.P. no 9**
**F-93230 Romainville (FR)**

(54) **Dérivés du 1,2,5,6-tétrahydropyridin-3-carboxaldéhyde oxime, leur procédé de préparation et leur application comme médicaments.**

(57) L'invention a pour objet les composés de formule (I) :

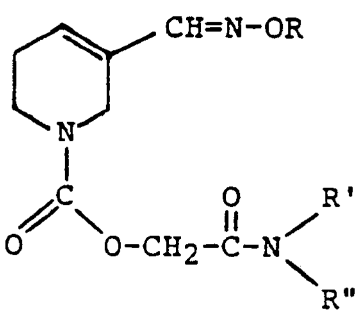

(I)

dans lesquels :
— R représente un atome d'hydrogène, un radical alkyle, alkényle ou alkynyle, linéaire, ramifié ou cyclique,
— R' et R" identiques ou différents l'un de l'autre représentent un atome d'hydrogène, un radical alkyle, alkényle ou alkynyle, linéaire, ramifié ou cyclique ou forment ensemble avec l'atome d'azote auquel ils sont liés un radical hétérocyclique.
Les composés de formule (I) présentent d'intéressantes propriétés pharmacologiques qui justifient leur utilisation comme médicament.

EP 0 452 170 A1

# NOUVEAUX DERIVES DU 1,2,5,6-TETRAHYDROPYRIDIN-3-CARBOXALDEHYDE OXIME, LEUR PROCEDE DE PREPARATION ET LEUR APPLICATION COMME MEDICAMENTS

La présente invention concerne de nouveaux dérivés du 1,2,5,6-tétrahydropyridin-3-carboxaldéhyde oxime, leur procédé de préparation et leur application comme médicament.

L'invention a pour objet les composés de formule (I) :

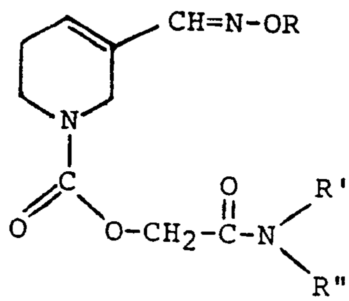

(I)

dans lesquels :

– R représente un atome d'hydrogène, un radical alkyle, alkényle ou alkynyle renfermant jusqu'à 8 atomes de carbone, linéaire, ramifié ou cyclique,

– R′ et R″ identiques ou différents l'un de l'autre représentent un atome d'hydrogène, un radical alkyle, alkényle ou alkynyle renfermant jusqu'à 8 atomes de carbone linéaire, ramifié ou cyclique ou forment ensemble avec l'atome d'azote auquel ils sont liés un radical hétérocyclique.

Lorsque R, R′ ou R″ représentent un radical alkyle saturé, linéaire ou ramifié, il s'agit de préférence d'un radical méthyle, éthyle, propyle, isopropyle, butyle, sec-butyle, isobutyle, pentyle, hexyle, tert-butyle, tert-pentyle, néopentyle ou hexyle.

Lorsque R, R′ ou R″ représentent un radical alkyle aliphatique insaturé, il s'agit de préférence d'un radical éthylénique comme, par exemple, d'un radical vinyle, allyle, 1,1-diméthylallyle, 2-buténylé, ou d'un radical acétylénique comme, par exemple, le radical éthynyle ou propynyle.

Lorsque R, R′ ou R″ représentent un radical alkyle cyclique, il s'agit de préférence d'un radical cyclopropyle, cyclobutyle, cyclopentyle, cyclohexyle, cyclopropylméthyle, cyclobutylméthyle, cyclopentylméthyle ou cyclohexylméthyle.

Lorsque R′ et R″ forment avec l'atome d'azote, auquel ils sont liés un hétérocycle, il peut s'agir d'un hétérocycle renfermant ce seul atome d'azote comme seul hétéroatome, il peut s'agir par exemple d'un cycle pipéridinyle ou pyrrolidinyle, il peut s'agir aussi d'un hétérocycle renfermant un deuxième hétéroatome, par exemple d'un hétérocycle renfermant un deuxième atome d'azote, ou encore d'un hétérocycle renfermant un atome d'oxygène ou de soufre, par exemple un cycle pipérazinyle ou morpholinyle éventuellement substitué par un radical alkyle renfermant jusqu'à 4 atomes de carbone.

Parmi les produits préférés de l'invention, on peut citer :

– les composés de formule (I) dans lesquels R représente un radical alkyle linéaire renfermant jusqu'à 8 atomes de carbone, par exemple un radical méthyle,

– les composés de formule (I) dans lesquels R′ et R″ identiques représentent un radical alkényle renfermant jusqu'à 4 atomes de carbone, par exemple un radical allyle,

– les produits de formule (I) dans lesquels les radicaux R′ et R″ forment avec l'atome d'azote auquel ils sont liés un radical pipéridinyle, pipérazinyle ou N-méthyl pipérazinyle, et notamment le produit de l'exemple 3.

Les composés de l'invention présentent de très intéressantes propriétés pharmacologiques et notamment une activité cholinomimétique par voie orale importante de longue durée d'action.

Il est bien connu que les troubles de l'apprentissage et de la mémoire chez les personnes âgées sont surtout reliés à un déficit du système cholinergique central, en particulier dans la démence sénile et la maladie d'Alzheimer.

Il est donc évident que les produits ayant une action cholinergique centrale puissent être employés dans le traitement thérapeutique de ces maladies (Bartus, R.I. Science 217, 408, 1982).

Il a été démontré que l'arécoline injectée par voie intraveineuse a un effet positif sur des patients ayant un déficit de la mémoire (Sitaram N. et al. Science 201, 274, 1978) (Christie J.E. et al. Brit. J. Psychiatry 138, 46, 1981).

Une limitation à l'emploi thérapeutique de l'arécoline est reliée au fait que ce produit a une très faible activité par voie orale et une courte durée d'action.

Les produits, objet de l'invention, ont démontré, après administration par voie orale, une activité cholinomimétique centrale supérieure à celle de l'arécoline et avec une plus longue durée d'action.

L'invention a donc pour objet les produits de l'invention en tant que médicaments, utiles notamment dans le traitement de la maladie d'Alzheimer ou de la démence sénile et également dans le traitement des troules de la mémoire.

L'invention a particulièrement pour objet en tant que médicaments, les composés préférés cités ci-dessus et notamment les composés des exemples.

L'invention a plus particulièrement pour objet en tant que médicament le produit de l'exemple 3.

La posologie usuelle est variable selon l'affection en cause, le sujet traité et la voie d'administration, elle peut être comprise entre 1 mg et 100 mg/jour, par exemple, entre 1 et 15 mg/jour en une ou plusieurs prises pour le produit de l'exemple 3 administré par voie orale.

La présente invention a également pour objet les compositions pharmaceutiques renfermant comme principe actif au moins un produit de formule (I). Les compositions pharmaceutiques de l'invention peuvent être solides ou liquides et se présenter sous les formes pharmaceutiques couramment utilisées en médecine humaine, comme, par exemple, les comprimés simples ou dragéifiés, les gélules, les granulés, les suppositoires, les préparations injectables ; elles sont préparées selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les produits de l'invention présentent l'avantage non négligeable d'être stables en milieu aqueux.

L'invention a également pour objet un procédé de préparation caractérisé en ce que l'on soumet un composé de formule (II) :

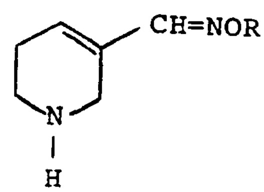

(II)

dans laquelle R conserve la même signification que précédemment, à l'action d'un composé de formule (III) :

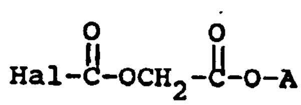

(III)

dans laquelle A représente un groupe protecteur de la fonction acide, et Hal représente un atome d'halogène, pour obtenir le composé de formule (IV) :

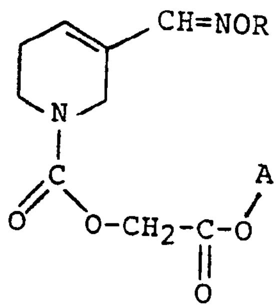

(IV)

que l'on soumet à l'action d'un agent de clivage du groupe protecteur, pour obtenir l'acide de formule (V) :

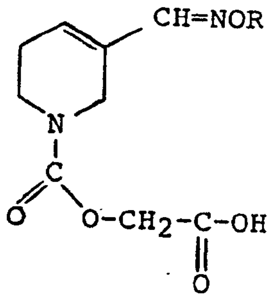

(V)

puis soumet un dérivé fonctionnel de cet acide (V) à l'action d'une amine de formule (VI) :

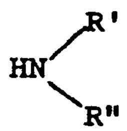

(VI)

dans laquelle R' et R" conservent la même signification que précédemment pour obtenir le composé de formule (I) correspondant.

Dans un mode de réalisation préféré A représente un radical benzyle, éventuellement substitué par un radical $NO_2$,

– Hal représente un atome de chlore,

– la réaction du composé de formule (II) avec le composé de formule (III) a lieu en présence d'une base comme par exemple la triéthylamine dans un solvant inerte comme par exemple le benzène ou le dichlorométhane,

– l'agent de clivage du groupe protecteur paranitrobenzyle est le sulfure de sodium en solution aqueuse à laquelle on ajoute un solvant organique, par exemple du tétrahydrofuranne,

– la réaction du composé de formule (V) avec le composé de formule (VI) a lieu après transformation de la fonction acide en chlorure, par action du chlorure de thionyl dans du chloroforme.

Les composés de formule (II) sont des produits connus décrits dans le brevet européen 239445.

Les composés de formule (III) sont des produits nouveaux qui peuvent être préparés comme indiqué ci-après dans la partie expérimentale. Cette préparation peut être résumée par le schéma réactionnel suivant :

AOH ---> AHal

Les produits AOH et notamment l'hydroxyacétate de 4-nitrobenzyle peuvent être préparés selon le procédé décrit dans CA 61, 4469g ; Izv. AKAD. NAUK. SSSR Ser. Khim (1964) pages 685-692.

Les exemples suivants illustrent l'invention sans toutefois la limiter.

**Preparation : Chlorocarbonyloxyacétate de 4-nitro benzyle**

On introduit dans 60 cm³ de benzène, 17,8 g de phosgène et 7,1 g d'hydroxy acétate de 4-nitrobenzyle (Izv Akad Nauk SSSR. Khim 1964 (4) 685-692, Chem. Abst. 61, 4469g) en suspension dans 150 cm³ de benzène. On agite 2 heures à température ambiante, amène à 7°/8°C et ajoute 5,16 cm³ de diazabicycloundécène. On maintient 1 heure à cette température, décante et concentre partiellement la phase surnageante sous pression réduite à 30°/35°C jusqu'à obtention d'environ 30 cm³ et utilise le produit tel que pour le stade suivant.

**Exemple 1 : 3-(méthoxyiminométhyl) 1,2,5,6-tétrahydro pyridine-1-carboxylate de (aminocarbonyl) méthyle**

Stade A : 3-(méthoxy imino méthyl) 1,2,5,6-tétrahydro pyridine 1-carboxylate de (4-nitrobenzyl) méthyle.

On introduit sous atmosphère inerte 1,66 g de 1,2,5,6-tétrahydropyridin-3-carboxaldéhyde O-méthyl oxime dans 30 cm³ de benzène, ajoute 1,65 cm³ de triéthylamine, refroidit à une température comprise entre 7° et 13°C et ajoute le milieu réactionnel obtenu à la préparation ci-dessus. On agite la suspension 1 heure à température ambiante et abandonne 16 heures. On lave le milieu réactionnel avec une solution aqueuse à 5% d'acide chlorhydrique, sépare la phase aqueuse que l'on extrait à l'acétate d'éthyle, réunit les phases organiques, sèche et évapore les solvants sous pression réduite. On chromatographie le résidu sur silice (éluant :

cyclohexane-acétate d'éthyle 3-2). Après cristallisation dans l'acétate d'éthyle-cyclohexane, on obtient 3,95 g de produit attendu.
F = 93-94°C.

| | | | | |
|---|---|---|---|---|
| Analyse : | $C_{17}H_{19}N_3O_7$ : 377,353 | | | |
| Calculé : | C% 54,11 | H% 5,08 | N% 11,14 | |
| Trouvé : | 54,28 | 5,16 | 10,88 | |

Stade B : acide [(3-méthoxyiminométhyl) 1,2,5,6-tétrahydropyridin-1-yl-carbonyl] oxy acétique.

On introduit 5,63 g de produit obtenu au stade A dans 130 cm³ de tétrahydrofuranne et 70 cm³ d'eau. On refroidit à 0°C, ajoute 3,58 g de sulfure de sodium nonahydraté dans 70 cm³ d'eau. On agite 1 heure à cette température, ajoute 11,3 cm³ d'acide chlorhydrique N, évapore le tétrahydrofuranne et extrait à l'acétate d'éthyle l'alcool p-nitrothiobenzylique. On acidifie avec de l'acide chlorhydrique 2N, extrait à l'acétate d'éthyle, sèche sur sulfate de sodium et évapore. Après cristallisation dans l'acétate d'éthyle-éther diisopropylique, on obtient 3,25 g de produit attendu. F = 133-134°C (décomposition).

| | | | |
|---|---|---|---|
| Analyse : | $C_{10}H_{14}N_2O_5$ : 242,231 | | |
| Calculé : | C% 49,59 | H% 5,83 | N% 11,57 |
| Trouvé : | 49,55 | 5,91 | 11,44 |

Stade C : Chlorure d'acide [[(3-méthoxyiminométhyl) 1,2,5,6-tétrahydro pyridin-1-yl-carbonyl] oxy] acétique

On chauffe 30 minutes au reflux 1,5 g de l'acide obtenu ci-dessus et 4,5 cm³ de chlorure de thionyle dans 15 cm³ de chloroforme. On élimine sous pression réduite l'excès de chlorure de thionyle et le solvant. On utilise l'huile résiduelle telle quelle pour le stade suivant.

Stade D : 3-(méthoxyiminométhyl) 1,2,5,6-tétrahydro pyridine-1-carboxylate de (aminocarbonyl) méthyle

On prépare de nouveau du chlorure d'acide [[(3-méthoxyiminométhyl) 1,2,5,6-tétrahydropyridin-1-yl carbonyl] oxy] acétique comme indiqué au stade C à partir de 1,1 g d'acide obtenu au stade B et 3,3 cm³ de chlorure de thionyle. On reprend l'huile résiduelle dans 10 cm³ de chloroforme et verse dans une solution comprenant 0,7 g d'ammoniac dans 20 cm³ de chloroforme. On agite la suspension pendant 1 heure et demie, lave à l'eau, sépare la phase aqueuse que l'on extrait à l'acétate d'éthyle, sèche et évapore. On chromatographie le résidu sur silice (éluant : chloroforme-méthanol 9-1) et obtient 0,65 g de produit attendu que l'on ajoute à 0,95 g de produit obtenu lors d'une préparation précédente et dilue dans du benzène. On obtient 1,45 g de produit attendu.
F = 131-133°C.

| | | | |
|---|---|---|---|
| Analyse : | $C_{10}H_{15}N_3O_4$ : 241,247 | | |
| Calculé : | C% 49,79 | H% 6,27 | N% 17,42 |
| Trouvé : | 49,64 | 6,25 | 17,17 |

**EXEMPLE 2 : 3-(méthoxyiminométhyl) 1,2,5,6-tétrahydropyridine-1-carboxylate de (diéthylaminocarbonyl) méthyle**

On opère comme au stade C de l'exemple 1 à partir de 1,5 g d'acide obtenu au stade B de l'exemple 1. On reprend l'huile résiduelle dans 20 cm³ de benzène, et verse dans une solution comprenant 1,3 cm³ de diéthylamine dans 20 cm³ de benzène (réaction exothermique). On agite 30 minutes à température ambiante, lave à l'eau, sépare la phase aqueuse que l'on extrait à l'acétate d'éthyle, réunit les phases organiques, les sèche et évapore à sec. On chromatographie le résidu sur silice (éluant : acétate d'éthyle) et récupère 1,35 g de produit attendu que l'on reprend dans l'hexane. F = 83-84°C ; insoluble dans $H_2O$, NaOH 2N, HCl 2N.

| | | | | |
|---|---|---|---|---|
| Analyse | : $C_{14}H_{23}N_3O_4$ | : 297,355 | | |
| Calculé | : | C% 56,55 | H% 7,80 | N% 14,13 |
| Trouvé | : | 56,51 | 7,82 | 14,02 |

**EXEMPLE 3 : 3-(méthoxyiminométhyl) 1,2,5,6-tétrahydropyridine-1-carboxylate de (di(2-propényl) aminocarbonyl) méthyle**

On opère comme au stade C de l'exemple 1 à partir de 1,5 g d'acide obtenu au stade B de l'exemple 1. On reprend l'huile résiduelle dans 20 cm³ de benzène et verse dans une solution comprenant 1,58 cm³ de diallylamine dans 20 cm³ de benzène. On poursuit la synthèse comme indiqué à l'exemple 2. Après chromatographie sur silice (éluant : acétate d'éthyle-n-hexane 7-3), on récupère 1,85 g d'huile jaune que l'on reprend dans l'hexane et précipite par l'éther. On recueille 1,75 g de produit cristallisé. F = 63,5-64°C ; insoluble dans $H_2O$, HCl 2N, NaOH 2N.

| | | | | |
|---|---|---|---|---|
| Analyse | : $C_{16}H_{23}N_3O_4$ | : 321,377 | | |
| Calculé | : | C% 59,80 | H% 7,21 | N% 13,08 |
| Trouvé | : | 59,58 | 7,18 | 12,99 |

**EXEMPLE 4 : 3-(méthoxyiminométhyl) 1,2,5,6-tétrahydropyridine-1-carboxylate de (1-pipéridinylcarbonyl) méthyle**

On opère comme au stade C de l'exemple 1 à partir de 2 g d'acide obtenu comme au stade B de l'exemple 1 et 6 cm³ de chlorure de thionyle. On reprend l'huile résiduelle dans 20 cm³ de benzène, verse dans une solution comprenant 1,65 cm³ de pipéridine (réaction exothermique). On poursuit la synthèse comme indiqué à l'exemple 2. Le résidu obtenu avant chromatographie est cristallisé dans l'éthanol aqueux après chromatographie sur charbon actif. On chromatographie le produit obtenu sur silice (éluant : acétate d'éthyle), concentre à sec, reprend dans l'éther et obtient 2,12 g de produit attendu. F = 93-94°C ; insoluble dans $H_2O$, HCl 2N, NaOH 2N.

| | | | | |
|---|---|---|---|---|
| Analyse | : $C_{15}H_{23}N_3O_4$ | : 309,366 | | |
| Calculé | : | C% 58,24 | H% 7,49 | N% 13,58 |
| Trouvé | : | 57,97 | 7,57 | 13,38 |

**EXEMPLE 5 : 3-(méthoxyiminométhyl) 1,2,5,6-tétrahydropyridine-1-carboxylate de ((4-méthyle-1-pipérazinyl) carbonylméthyle**

On opère comme au stade C de l'exemple 1 à partir de 1,45 g d'acide obtenu comme au stade B de l'exemple 1 et 4,4 cm³ de chlorure de thionyle. On reprend l'huile résiduelle dans 20 cm³ de benzène, verse dans une solution comprenant 1,34 cm³ de N-méthyl pipérazine dans 20 cm³ de benzène et agite 1 heure et demie à température ambiante. On lave à l'eau, sépare la phase aqueuse, la neutralise à l'aide de bicarbonate de sodium à 5% et l'extrait à l'acétate d'éthyle. On chromatographie sur silice (éluant : chloroforme-méthanol 9-1) et obtient 1,45 g d'huile que l'on cristallise par précipitation dans le n-hexane et obtient 1,25 g de produit attendu. F = 104-105°C ; soluble dans l'eau, HCl 2N, insoluble dans NaOH 2N.

| | | | | |
|---|---|---|---|---|
| Analyse | : $C_{15}H_{24}N_4O_4$ | : 324,381 | | |
| Calculé | : | C% 55,54 | H% 7,46 | N% 17,27 |
| Trouvé | : | 55,28 | 7,35 | 17,07 |

**EXEMPLE 6 :**

On a préparé des comprimés correspondant à la formulation suivante :

- Composé de l'exemple 3 ........................ 50 mg
- Excipient q.s. pour un comprimé terminé à ..... 300 mg

(Détail de l'excipient : lactose, amidon de blé, amidon traité, amidon de riz, talc, stéarate de magnésium).

**EXEMPLE 7 :**

On a préparé des gélules correspondant à la formulation suivante :

- Produit de l'exemple 3 ........................ 60 mg
- Excipient q.s. pour une gélule terminée à ..... 300 mg

(Détail de l'excipient : talc, stéarate de magnésium, aérosil).

**ETUDE PHARMACOLOGIQUE**

**Toxicité aigüe.**

L'essai est réalisé sur des souris mâles ($CD_1$) Charles Rivers) de 22 à 24 g, à jeun depuis 16 heures. On administre les produits par voie orale à la dose de 1000, 500, 250 et 125 mg/kg. On note la mortalité pendant les 7 jours suivant le traitement.

Produit de l'exemple 3 $DL_{50}$ = 500 mg/kg
Arécoline, HBr $DL_{50}$ = 600 mg/kg

**Test de l'iléon isolé de cobaye**

On prélève des fragments d'iléon de cobayes tués par décapitation. L'iléon isolé est placé dans 10 $cm^3$ de solution de Tyrode à 37°C et aéré par un mélange d'oxygène (95%) et de gaz carbonique (5%). Les contractions dues aux produits sont enregistrées à l'aide d'un capteur relié à un polygraphe. Les produits à tester sont ajoutés, aux concentrations comprises entre $1.10^{-3}$M et $1.10^{-8}$M/l.

Les produits présentant un effet contracturant sont testés vis à vis de l'atropine et de l'hexaméthonium pour établir si l'activité est de type "muscarinique" ou "nicotinique".

L'activité agoniste est exprimée en $pD_2$ (logarithme négatif de la dose qui produit 50% de l'effet maximum).

Produit de l'exemple 3 $pD_2 < 4$
Arécoline, HBr $pD_2 = 6,90$

**Activité diarrhéique.**

Le test est réalisé sur des souris mâles ($CD_1$ Charles Rivers) pesant 25 à 30 g, à jeun depuis 6 heures. Le produit dissous à 5% dans du méthocel est administré par voie orale, au moyen d'une sonde oesophagienne.

Des animaux témoins ne reçoivent que l'excipient.

Après traitement, les animaux sont mis séparément dans des cages dont le fond est recouvert de papier buvard et sont mis en observation pendant 30, 60, 120 et 180 minutes.

Les feuilles de papier absorbant sont changées après chaque observation.

La consistance des fèces est évaluée selon la méthode de Randall et Baruth (Arch. Int. Pharmacodyn. 220, 94, 1976) en suivant l'échelle des valeurs suivantes :

0 : consistance ferme,
1 : fèces légèrement molles avec ou sans auréole humide,
2 : fèces légèrement molles avec présence d'un cercle humide bien défini,
3 : fèces molles avec présence d'un grand cercle humide,

4 : fèces sans consistance avec présence d'un très grand cercle humide.

Pour chaque produit, on a noté la dose qui provoque une diarrhée chez 50% des animaux selon la méthode de Miller et Tainter (Proc. Soc. Exp. Biol. Med., 57, 261, 1944).

Produit de l'exemple 3 $DE_{50} > 50$

Arécoline, HBr $DE_{50}$ 35

**Activité hypothermique.**

Le test est réalisé sur des souris mâles ($CD_1$ Charles Rivers) pesant 25 à 30 g, à jeun depuis 6 heures.

La température du corps est notée au moyen d'un thermocouple placé dans le rectum à environ 1,5 cm et relié à un enregistreur de température électrique.

Les produits sont administrés par voie orale ou sous-cutanée et les températures sont notées à l'instant 0 et 30 minutes, 1 heure, 2 heures et 2 heures et demie après traitement.

On évalue le degré d'hypothermie comme la différence entre les animaux traités et les témoins et on détermine la dose nécessaire pour réduire de 1°c la température du corps.

| Exemple | Dose efficace (-1°C en mg/kg) | |
|---|---|---|
| | V.O | V.SC |
| 3 | 9 | 8 |
| Arécoline, HBr | 190 | 3 |

On détermine la durée d'action des produits en utilisant des doses capables de réduire la température de 1,5°c à 1,7°C.

**Variations de la température du corps (°C)**

| Exemple | dose mg/kg | administration | temps en minute de traitement 0 | 30 | 60 | 120 | 180 |
|---|---|---|---|---|---|---|---|
| 3 | 15 | os | +0,1 | -1,3** | -1,7** | -0,5 | +0,1 |
| | 12 | sc | ±0 | -0,9** | -1,6** | -0,6 | ±0 |
| Arécoline, HBr | 3,5 | sc | -0,1 | -1,5** | -0,1 | +0,2 | +0,2 |

** Valeurs différentes des témoins d'une manière significative ($p < 0,01$).

**Revendications**

1.- Les composés de formule (I) :

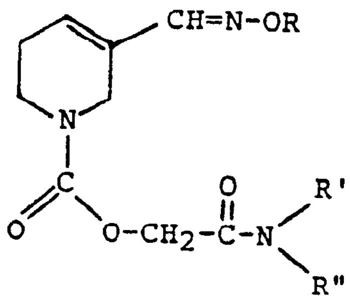

(I)

dans lesquels :

– R représente un atome d'hydrogène, un radical alkyle, alkényle ou alkynyle renfermant jusqu'à 8 atomes de carbone, linéaire, ramifié ou cyclique,

– R′ et R″ identiques ou différents l'un de l'autre représentent un atome d'hydrogène, un radical alkyle, alkényle ou alkynyle renfermant jusqu'à 8 atomes de carbone linéaire, ramifié ou cyclique ou forment ensemble avec l'atome d'azote auquel ils sont liés un radical hétérocyclique.

**2.-** Les composés de formule (I) définis à la revendication 1, dans lesquels R représente un radical alkyle linéaire renfermant jusqu'à 8 atomes de carbone.

**3.-** Les composés de formule (I) définis à la revendication 2, dans lesquels R représente un radical méthyle.

**4.-** Les composés de formule (I) définis à l'une quelconque des revendications 1 à 3, dans lesquels R′ et R″ identiques représentent un radical alkényle renfermant jusqu'à 4 atomes de carbone.

**5.-** Les composés de formule (I) tels que définis à la revendication 4, dans lesquels R′ et R″ représentent un radical allyle.

**6.-** Les composés de formule (I) définis à l'une quelconque des revendications 1 à 3, dans lesquels les radicaux R et R′ forment avec l'atome d'azote auquel ils sont liés un radical pipéridinyle, pipérazinyle ou N-méthyl pipérazinyle.

**7.-** Le composé de formule (I) défini à la revendication 5 dont le nom suit :

– le 3-(méthoxyiminométhyl) 1,2,5,6-tétrahydro-pyridine-1-carboxylate de (di(2-propényl) aminocarbonyl) méthyle.

**8.-** A titre de médicaments, les composés de formule (I) définis à l'une quelconque des revendications 1 à 6.

**9.-** A titre de médicaments le composé de la revendication 7.

**10.-** Les compositions pharmaceutiques renfermant comme principe actif au moins un médicament défini à la revendication 8 ou 9.

**11.-** Procédé de préparation des composés de formule (I) défini à l'une quelconque des revendications 1 à 7, caractérisé en ce que l'on soumet un composé de formule (II) :

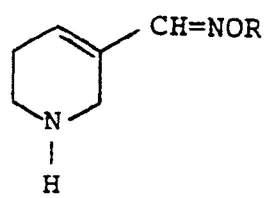

(II)

dans laquelle R conserve la même signification que précédemment, à l'action d'un composé de formule (III) :

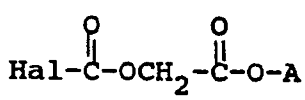

(III)

dans laquelle A représente un groupe protecteur de la fonction acide, et Hal représente un atome d'halogène, pour obtenir le composé de formule (IV) :

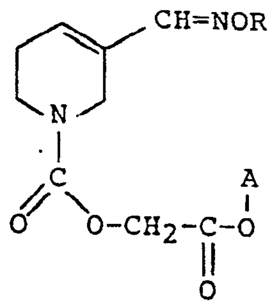

(IV)

que l'on soumet à l'action d'un agent de clivage du groupe protecteur, pour obtenir l'acide de formule (V) :

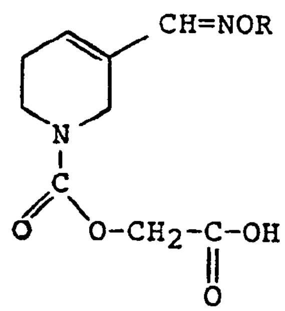

(V)

puis soumet un dérivé fonctionnel de cet acide (V) à l'action d'une amine de formule (VI) :

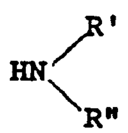

(VI)

dans laquelle R' et R" conservent la même signification que précédemment pour obtenir le composé de formule (I) correspondant.

**12.**- A titre de produits industriels nouveaux, les composés de formule (III), (IV), et (V) tels que définis à la revendication 11.

**Revendications pour l'Etat contractant suivant: ES**

**1.**- Procédé pour préparer des composés de formule (I) :

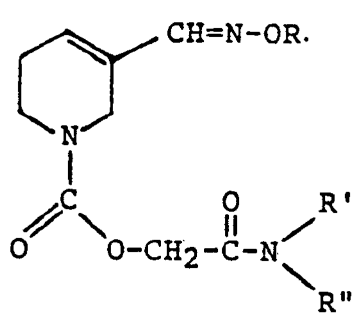

(I)

dans lesquels :

- R représente un atome d'hydrogène, un radical alkyle, alkényle ou alkynyle renfermant jusqu'à 8 atomes de carbone, linéaire, ramifié ou cyclique,
- R' et R" identiques ou différents l'un de l'autre représentent un atome d'hydrogène, un radical alkyle, alkényle ou alkynyle renfermant jusqu'à 8 atomes de carbone linéaire, ramifié ou cyclique ou forment ensemble avec l'atome d'azote auquel ils sont liés un radical hétérocyclique, caractérisé en ce que l'on soumet un composé de formule (II) :

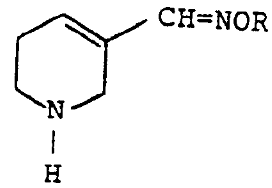

(II)

dans laquelle R conserve la même signification que précédemment, à l'action d'un composé de formule (III) :

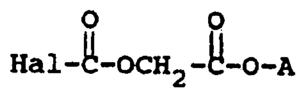

(III)

dans laquelle A représente un groupe protecteur de la fonction acide, et Hal représente un atome d'halogène, pour obtenir le composé de formule (IV) :

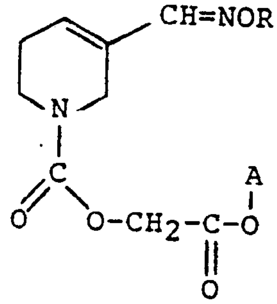

(IV)

que l'on soumet à l'action d'un agent de clivage du groupe protecteur, pour obtenir l'acide de formule (V) :

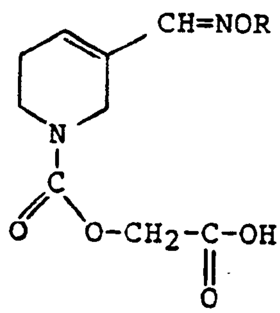

(V)

puis soumet un dérivé fonctionnel de cet acide (V) à l'action d'une amine de formule (VI) :

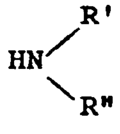

(VI)

dans laquelle R′ et R″ conservent la même signification que précédemment pour obtenir le composé de formule (I) correspondant.

**2.**- Procédé selon la revendication 1, caractérisé en ce que l'on utilise au départ un composé de formule (II) dans laquelle R représente un radical alkyle linéaire renfermant jusqu'à 8 atomes de carbone.

**3.**- Procédé selon la revendication 2, caractérisé en ce que l'on utilise au départ un composé de formule (II) dans laquelle R représente un radical méthyle.

**4.**- Procédé selon la revendication 1, caractérisé en ce que l'on soumet un dérivé de l'acide de formule (V) à l'action d'une amine de formule (VI) dans laquelle R′ et R″ identiques représentent un radical alkényle

renfermant jusqu'à 4 atomes de carbone.

**5.**- Procédé selon la revendication 1, caractérisé en ce que l'on soumet un dérivé de l'acide de formule (V) à l'action d'une amine de formule (VI) dans laquelle R′ et R″ représentent un radical allyle.

**6.**- Procédé selon la revendication 1, caractérisé en ce que l'on soumet un dérivé de l'acide de formule (V) à l'action d'une amine de formule (VI) dans laquelle R et R′ forment avec l'atome d'azote auquel ils sont liés un radical pipéridinyle, pipérazinyle ou N-méthyl pipérazinyle.

**Revendications pour l'Etat contractant suivant: GR**

**1.**- Procédé pour préparer des composés de formule (I) :

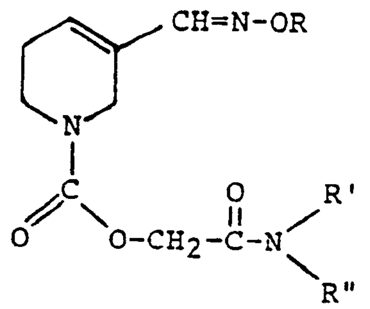

(I)

dans lesquels :

– R représente un atome d'hydrogène, un radical alkyle, alkényle ou alkynyle renfermant jusqu'à 8 atomes de carbone, linéaire, ramifié ou cyclique,

– R′ et R″ identiques ou différents l'un de l'autre représentent un atome d'hydrogène, un radical alkyle, alkényle ou alkynyle renfermant jusqu'à 8 atomes de carbone linéaire, ramifié ou cyclique ou forment ensemble avec l'atome d'azote auquel ils sont liés un radical hétérocyclique, caractérisé en ce que l'on soumet un composé de formule (II) :

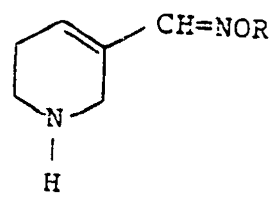

(II)

dans laquelle R conserve la même signification que précédemment, à l'action d'un composé de formule (III) :

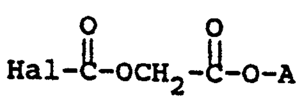

(III)

dans laquelle A représente un groupe protecteur de la fonction acide, et Hal représente un atome d'halogène, pour obtenir le composé de formule (IV) :

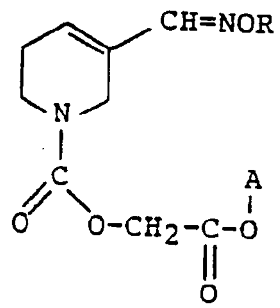

(IV)

que l'on soumet à l'action d'un agent de clivage du groupe protecteur, pour obtenir l'acide de formule (V) :

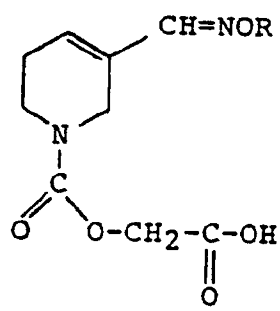

(V)

puis soumet un dérivé fonctionnel de cet acide (V) à l'action d'une amine de formule (VI) :

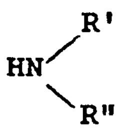

(VI)

dans laquelle R′ et R″ conservent la même signification que précédemment pour obtenir le composé de formule (I) correspondant.

**2.**- Procédé selon la revendication 1, caractérisé en ce que l'on utilise au départ un composé de formule (II) dans laquelle R représente un radical alkyle linéaire renfermant jusqu'à 8 atomes de carbone.

**3.**- Procédé selon la revendication 2, caractérisé en ce que l'on utilise au départ un composé de formule (II) dans laquelle R représente un radical méthyle.

**4.**- Procédé selon la revendication 1, caractérisé en ce que l'on soumet un dérivé de l'acide de formule (V) à l'action d'une amine de formule (VI) dans laquelle R′ et R″ identiques représentent un radical alkényle renfermant jusqu'à 4 atomes de carbone.

**5.**- Procédé selon la revendication 1, caractérisé en ce que l'on soumet un dérivé de l'acide de formule (V) à l'action d'une amine de formule (VI) dans laquelle R′ et R″ représentent un radical allyle.

**6.**- Procédé selon la revendication 1, caractérisé en ce que l'on soumet un dérivé de l'acide de formule (V) à l'action d'une amine de formule (VI) dans laquelle R et R′ forment avec l'atome d'azote auquel ils sont liés un radical pipéridinyle, pipérazinyle ou N-méthyl pipérazinyle.

**7.**- Procédé selon la revendication 5, caractérisé en ce que l'on utilise au départ des composés choisis de manière telle que l'on prépare :

– le 3-(méthoxyiminométhyl) 1,2,5,6-tétrahydro-pyridine-1-carboxylate de (di(2-propényl) aminocarbonyl) méthyle.

**8.**- A titre de produits industriels nouveaux, les composés de formule (III), (IV), et (V) tels que définis à la revendication 1.

**9.**- Procédé de préparation de compositions pharmaceutiques, caractérisé en ce que l'on met à titre de principe actif, l'un au moins des composés de formule (I) telle que définie à la revendication 1 sous une forme destinée à cet usage.

**10.**- Procédé de préparation de compositions pharmaceutiques, caractérisé en ce que l'on met à titre de principe actif, l'un au moins des composés de formule (I) telle que définie à l'une quelconque des revendications 2 à 7 sous une forme destinée à cet usage.